(19) Europäisches Patentamt
European Patent Office
Office européen des brevets

(11) Publication number: **0 257 457 B1**

(12) # EUROPEAN PATENT SPECIFICATION

(45) Date of publication of the patent specification: **31.10.90**

(21) Application number: **87111688.5**

(22) Date of filing: **12.08.87**

(51) Int. Cl.[5]: **C07C 69/22,** C07D 239/34, C07D 239/26, C07D 213/30, C07D 237/14, C09K 19/12, C09K 19/34, G02F 1/13

(54) **Optically active 2-methyl 1,3-propanediol derivatives, liquid crystal compositions comprising them and optical switching element.**

(30) Priority: **12.08.86 JP 189127/86**

(43) Date of publication of application: **02.03.88 Bulletin 88/9**

(45) Publication of the grant of the patent: **31.10.90 Bulletin 90/44**

(84) Designated Contracting States: **CH DE FR GB LI**

(56) References cited:
**EP-A- 0 131 373**
**EP-A- 0 168 043**

(73) Proprietor: **Chisso Corporation, 6-32, Nakanoshima 3-chome Kita-ku, Osaka-shi Osaka-fu(JP)**

(72) Inventor: **Saito, Shinichi, 10-1, Otsutomo-cho Kanazawa-ku, Yokohama-shi Kanagawa(JP)**
Inventor: **Ohno, Kouji, 10-3, Otsutomo-cho Kanazawa-ku, Yokohama-shi Kanagawa(JP)**
Inventor: **Inukai, Takashi, 4-46, Mori 3-chome Isogo-ku, Yokohama-shi Kanagawa(JP)**
Inventor: **Inoue, Hiromichi, 10-2, Otsutomo-cho Kanazawa-ku, Yokohama-shi Kanagawa(JP)**
Inventor: **Miyazawa, Kazutoshi, 12-14, Mutsuura 2-chome Kanazawa-ku, Yokohama-shi Kanagawa(JP)**

(74) Representative: **Hansen, Bernd, Dr.rer.nat. et al, Hoffmann, Eitle & Partner Patentanwälte Arabellastrasse 4 Postfach 81 04 20, D-8000 München 81(DE)**

EP 0 257 457 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

FIELD OF THE INVENTION

This invention relates to a novel organic compound, to a liquid crystal composition comprising the compound, and to an optical switching element utilizing as a liquid crystal the composition. More particularly, it relates to an organic compound having an optically active group which is useful as a component of a ferroelectric liquid crystal composition, to a ferroelectric liquid crystal composition comprising the compound, and to an optical switching element utilizing as a liquid crystal the composition.

BACKGROUND OF THE INVENTION

The TN (twisted nematic) type liquid crystal display mode is now most commonly used as a liquid crystal display element. The TN type liquid crystal display mode has many advantages, such as a low driving voltage and a small electric power consumption. In respect of response rate, however, the TN type liquid crystal display element is inferior to emissive type display elements, e.g., a cathode ray tube, electroluminescence, and plasma display. New TN type liquid crystal display elements in which the twisted angle is increased from 90° as used in the conventional TN type liquid crystal display element to a range of from 180° to 270° have been developed. These new TN type liquid crystal display elements, however, are still inferior in response rate. Although various attempts to improve the response rate have been made, a TN type liquid crystal display element sufficiently satisfactory in response rate has not yet been obtained.

It is reported in Clark et al., Applied Phys. Lett., Vol. 36, 899 (1980) that in a new display mode utilizing ferroelectric liquid crystals which have been extensively studied in recent years, there is a great possibility that the response rate would be markedly improved. This display mode utilizes a chiral smectic phase, such as a chiral smectic C phase (hereinafter referred to as SC*) which exhibits ferroelectric properties. It is known that not only the SC* phase but also phases, such as chiral smectic F, G, H, and I phases, exhibit ferroelectric properties. A ferroelectric liquid crystal material to be applied to practical ferroelectric liquid crystal display elements is required to have various characteristics. At present, these requirements are not met by a single compound so that several liquid crystal compounds or non-liquid crystal compounds should be mixed to prepare a desired ferroelectric liquid crystal composition.

SUMMARY OF THE INVENTION

As a result of extensive and intensive investigations, the inventors have found a compound having a property to increase a spontaneous polarization value (PS) that is one of the important characteristics required for the ferroelectric liquid crystal composition.

That is, the present invention relates to an optically active 2-methyl-1,3-propanediol derivative represented by formula (I)

$$R^1\text{-A-B-O-}CH_2\overset{\overset{\displaystyle CH_3}{|}}{\underset{*}{C}}HCH_2\text{-O-}R^2 \qquad (I)$$

wherein $R^1$ represents an alkyl group having 1 to 20 carbon atoms or an alkoxy group having from 1 to 20 carbon atoms, $R^2$ represents an alkyl group having from 1 to 15 carbon atoms, an alkanoyl group having from 2 to 15 carbon atoms, an alkoxycarbonyl group having from 2 to 15 carbon atoms, an alkoxyalkyl group having from 2 to 15 carbon atoms, an alkoxyalkanoyl group having from 3 to 15 carbon atoms, a halogenated alkyl group having from 1 to 15 carbon atoms or a halogenated alkanoyl group having from 2 to 15 carbon atoms, and the alkyl moiety in $R^1$ and $R^2$ is straight or branched; A and B each represents

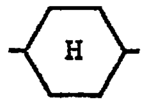, 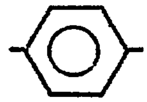, 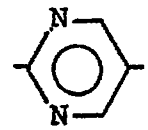,

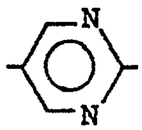, 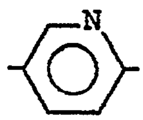, 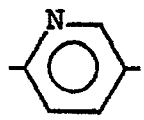, 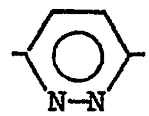, 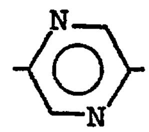,

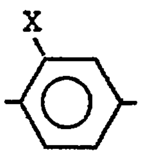 or 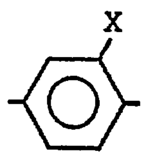,

wherein X represents a halogen atom, a cyano group or a trifluoromethyl group; and the asterisk mark * indicates that the carbon atom provided with * is an asymmetrical carbon atom, a liquid crystal composition comprising at least one of the compounds represented by formula (I), and particularly a ferroelectric liquid crystal composition, and an optical switching element using as a liquid crystal the liquid crystal composition comprising at least one of the compounds represented by formula (I).

DETAILED DESCRIPTION OF THE INVENTION

In formula (I), $R^1$ preferably represents an alkyl group having from 4 to 16 carbon atoms or an alkoxy group having from 4 to 16 carbon atoms.

$R^2$ preferably represents an alkyl group, an alkanoyl group or an alkoxyalkanoyl group, and more preferably contains up to 10 carbon atoms. The group represented by $R^2$ may be cptically active or inactive.

A combination of the rings A and B preferably includes

includes 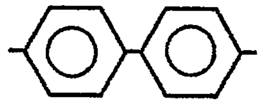, 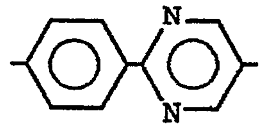, 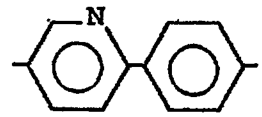

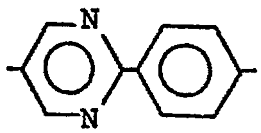, 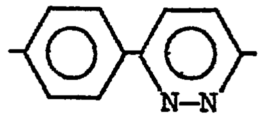, 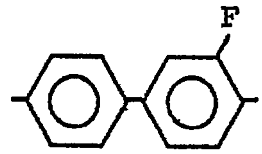,

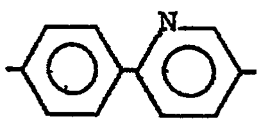, 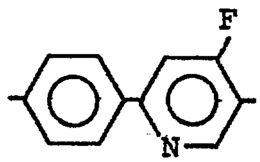, 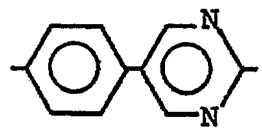,

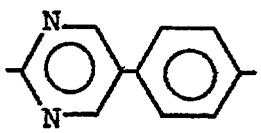, 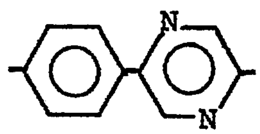, 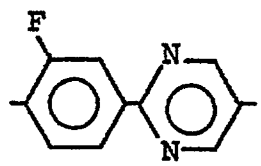,

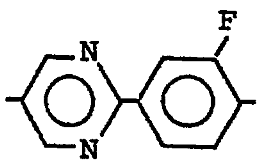, 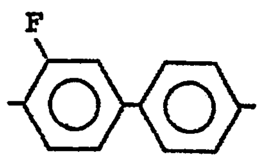, 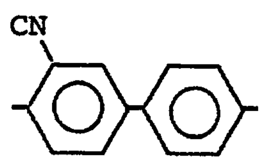,

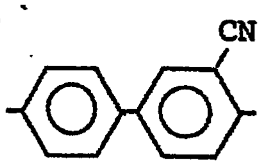, and 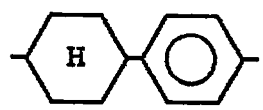,

with the first six combinations being more preferred.

Typical examples of the compounds represented by formula (I) are shown in Table 1 below along with their melting points.

TABLE 1

| Compound No. | $R^1$ | -A-B- | $R^2$ | Absolute Configuration | Melting Point (°C) | Remark |
|---|---|---|---|---|---|---|
| 1 | $n-C_8H_{17}O$ | 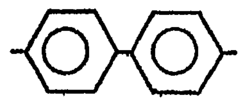 | 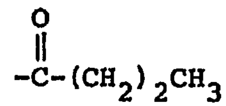 | S | 73.6-74.4 | Example 1 |
| 2 | $n-C_8H_{17}O$ | 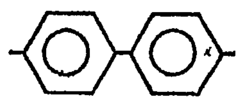 | 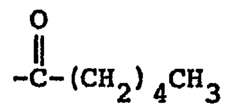 | S | 62.4 | |
| 3 | $n-C_8H_{17}O$ | 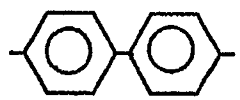 | 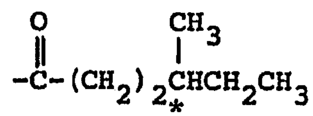 | S,S | 57.6 | |
| 4 | $n-C_7H_{15}O$ | 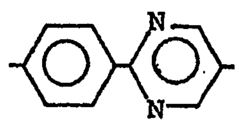 | 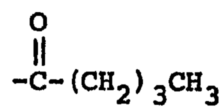 | R | 39.4 | |
| 5 | $n-C_7H_{15}O$ | 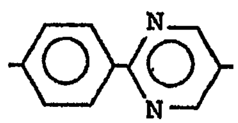 | 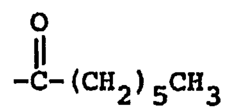 | R | 36.0 | |
| 6 | $n-C_7H_{15}O$ | 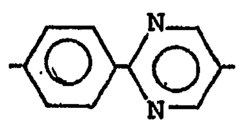 | 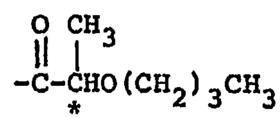 | R,S | 20.0 | |
| 7 | $n-C_7H_{15}O$ | 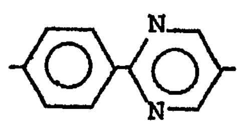 | $-(CH_2)_3CH_3$ | S | 22.2 | |

(cont'd)

| Compound No. | $R^1$ | -A-B- | $R^2$ | Absolute Configuration | Melting Point (°C) | Remark |
|---|---|---|---|---|---|---|
| 8 | $n-C_8H_{17}$ | 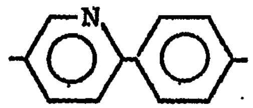 | $-\overset{O}{\overset{\|}{C}}-(CH_2)_2CH_3$ | R | 55.8 | |
| 9 | $n-C_8H_{17}$ | 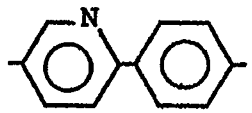 | $-\overset{O}{\overset{\|}{C}}-(CH_2)_4CH_3$ | R | 57.4 | |
| 10 | $n-C_8H_{17}$ | 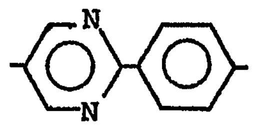 | $-\overset{O}{\overset{\|}{C}}-(CH_2)_2CH_3$ | R | 30.0 | |
| 11 | $n-C_8H_{17}$ | 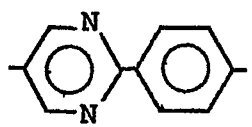 | $-\overset{O}{\overset{\|}{C}}-(CH_2)_3CH_3$ | R | 31.8 | |
| 12 | $n-C_8H_{17}$ | 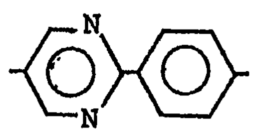 | $-\overset{O}{\overset{\|}{C}}-(CH_2)_3\underset{*}{\overset{CH_3}{\overset{\|}{C}}}HCH_2CH_3$ | R,S | 38.8 | |
| 13 | $n-C_8H_{17}O$ | 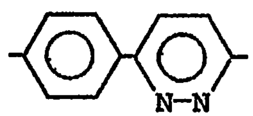 | $-\overset{O}{\overset{\|}{C}}-(CH_2)_3CH_3$ | R | 59.4 | |

(cont'd)

| Compound No. | $R^1$ | -A-B- | $R^2$ | Absolute Configuration | Melting Point (°C) | Remark |
|---|---|---|---|---|---|---|
| 14 | $n-C_8H_{17}O$ | 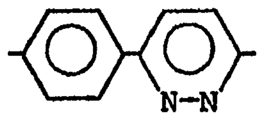 | 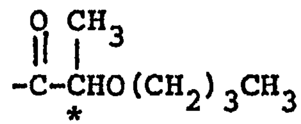 | R,S | 51.9 | |
| 15 | $n-C_8H_{17}O$ | 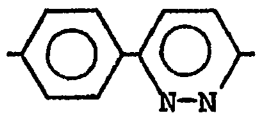 | 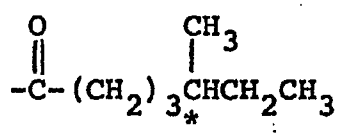 | R,S | 53.5 | |
| 16 | $n-C_{12}H_{25}$ | 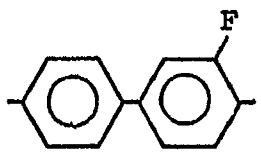 | 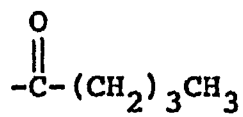 | R | 37.3 | |
| 17 | $n-C_{12}H_{25}$ | 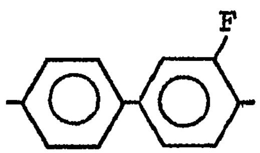 | 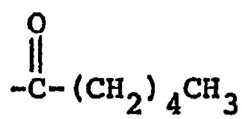 | R | 36.4 | |

Although many of the compounds according to the present invention have no liquid crystal properties by themselves, they provide ferroelectric liquid crystal compositions having high PS when combined with non-chiral smectic liquid crystal compounds or chiral smectic liquid crystal compounds. For example, a liquid crystal composition exhibiting a non-chiral smectic C phase which does not show spontaneous polarization becomes a ferroelectric liquid crystal composition on addition of 20% by weight of one of the compounds according to the present invention. The resulting composition has a high PS and a short response time. Further, addition of the compound of the present invention to a ferroelectric liquid crystal composition having a small PS and a long response time increases the PS and reduces the response time. Thus, the compounds of the present invention are very suitable as a constituting component of ferroelectric liquid crystal composition.

The non-chiral smectic liquid crystal compounds to be used in combination with the compounds of formula (I) include 5-alkyl-2-(4′-alkoxyphenyl)pyrimidine compounds, e.g., 5-octyl-2-(4′-octyloxyphenyl)pyrimidine, 5-octyl-2-(4′-nonyloxyphenyl)pyrimidine, 5-octyl-2-(4′-decyloxyphenyl)pyrimidine, etc.; 5-alkyl-2-(4′-alkoxyphenyl)pyridine compounds, e.g., 5-heptyl-2-(4′-octyloxy phenyl)pyridine, 5-octyl-2-(4′-octyloxyphenyl)pyridine, 5-heptyl-2-(4′-nonyloxyphenyl)pyridine, 5-nonyl-2-(4′-nonyloxyphenyl)pyridine, etc.; 5-alkyl-2-(4′-alkyl-4-biphenylyl)pyrimidine compounds, e.g., 5-octyl-2-(4′-hexyl-4-biphenylyl)pyrimidine, 5-heptyl-2-(4′-pentyl-4-biphenylyl)pyrimidine, 5-hexyl-2-(4′-octyl-4-biphenylyl)pyrimidine, etc.; 4-alkoxyphenyl-4-alkoxybenzoate compounds, e.g., 4-octyloxyphenyl 4-octyloxybenzoate, 4-nonyloxyphenyl 4-octyloxybenzoate, 4-decyloxyphenyl 4-nonyloxybenzoate, etc.; and the like.

The chiral smectic liquid crystal compounds to be used in combination with the compounds of formula (I) include optically active phenyl benzoate compounds, e.g., 4-(2′-methylbutyloxy)phenyl 4-octyloxybenzoate, 4-(6′-methyloctyloxy)phenyl 4-nonyloxybenzoate, etc.; optically active 5-alkylphenylpyrimidine compounds, e.g., 5-octyl-2-[4′-(6″-methyloctyloxy)phenyl]pyrimidine, 5-nonyl-2-[4′-(5″-methylheptyloxy)phenyl]pyrimidine, etc.; and optically active 5-alkylphenylpyridine compounds, e.g., 5-heptyl-2-[4′-(6″-methyloctyloxy)phenyl]pyridine, 5-octyl-2-[4′-(5″-methylheptyloxy)phenyl]pyridine, etc.

Since the compounds of formula (I) have an optically active carbon atom, they induce a twisted structure when added to a nematic liquid crystal. A nematic liquid crystal having the twisted structure, i.e., a chiral nematic liquid crystal, does not produce so-called reverse domains in a TN type liquid crystal display element so that the compounds of formula (I) can be used as an agents for preventing reverse domain formation.

The compounds of formula (I) can be synthesized through, for example, the following reaction route:

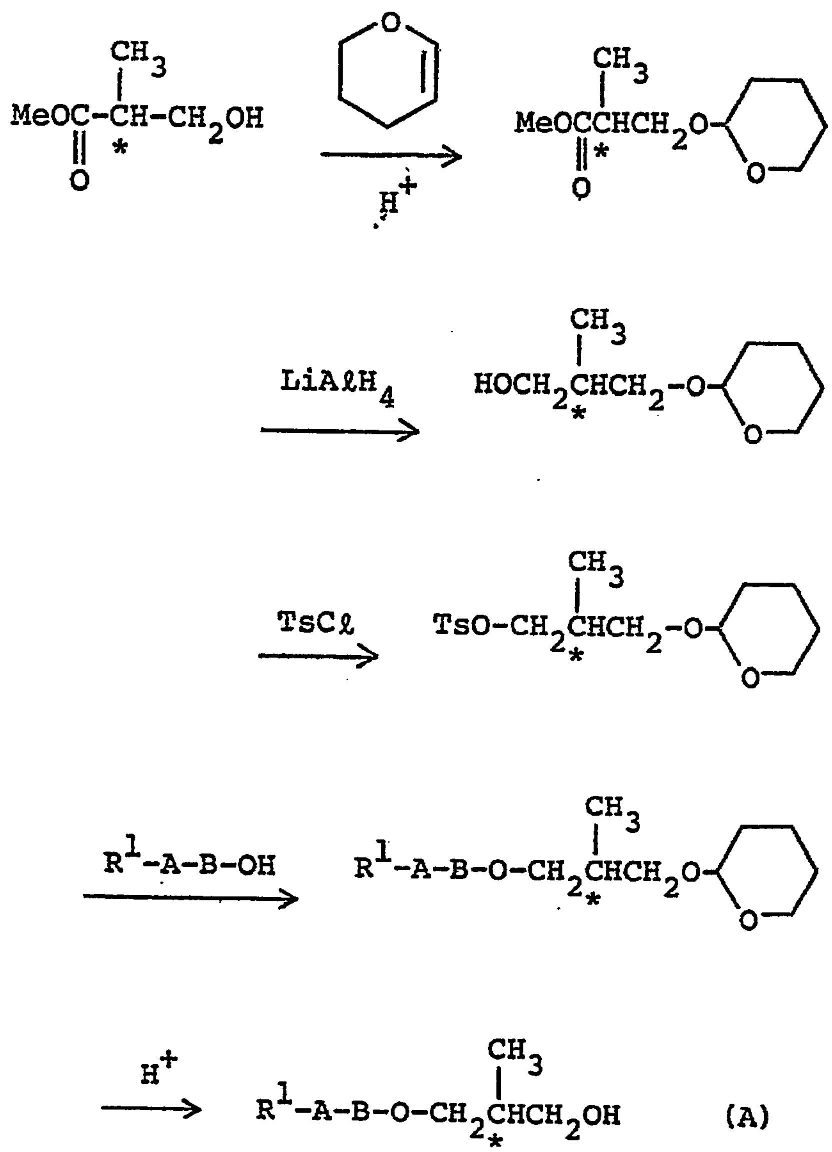


(A)

When $R^2$ is an alkyl group, an alkoxyalkyl group or a halogenated alkyl group,

$$\text{Compound (A)} \xrightarrow[\text{alkali}]{R^2L} \text{(I)}$$

When $R^2$ is an alkanoyl group, an alkoxyalkanoyl group or a halogenated alkanoyl group,

$$\text{Compound (A)} \xrightarrow[\text{dehydrating agent}]{R^2OH} \text{(I)}$$

When $R^2$ is an alkoxycarbonyl group,

$$\text{Compound (A)} \xrightarrow{R^2C\ell} \text{(I)}$$

wherein $R^1$, $R^2$, A, and B are as defined above; Ts represents a p-toluenesulfonyl group; and L represents a releasable group, such as a p-toluenesulfonyl group, a methanesulfonyl group, an iodine atom, and a bromine atom.

The present invention will now be illustrated in greater detail by way of the following examples, but it should be understood that the present invention is not limited thereto. In these examples, all the percents are by weight unless otherwise indicated.

EXAMPLE 1

Preparation of S-4′-Octyloxy-4-(2-methyl-3-butanoyloxypropoxy)biphenyl [the compound of formula (I) wherein $R^1$: an octyloxy group; $R^2$: a propyl group; and -A-B-:

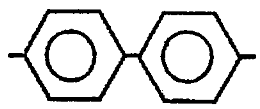
]

25 g of R-methyl 2-methyl-3-hydroxy-propionate, 21 g of 3,4-dihydropyran, 2 mℓ of concentrated hydrochloric acid, and 50 mℓ of anhydrous dichloromethane were mixed under ice-cooling, and the mixture was allowed to stand overnight. The mixture was washed with a 5% aqueous solution of sodium hydrogencarbonate and dried over magnesium sulfate. The solvent was removed by distillation, and the residue was distilled under reduced pressure to obtain 40 g of R-methyl 2-methyl-3-(2-tetrahydropyranyloxy)propionate having a boiling point of from 92 to 96°C/5 mmHg.

In 50 mℓ of tetrahydrofuran (THF) was suspended 7 g of lithium aluminum hydride, and 200 mℓ of a THF solution containing the whole quantity of the above prepared R-methyl 2-methyl-3-(2-tetrahydropyranyloxy)propionate was added to the suspension dropwise under cooling. After completion of the reaction, water and a 2N aqueous solution of sodium hydroxide were added to the reaction mixture, and any inorganic substance was separated by filtration. The filtrate was dried over magnesium sulfate, and the solvent was removed by distillation. The residue was distilled under reduced pressure to obtain 36 g of S-2-methyl-3-(2-tetrahydropyranyloxy)propan-1-ol having a boiling point of 68 to 75°C/1.5 mmHg.

In 500 mℓ of pyridine was dissolved 15 g of the resulting product, and 100 mℓ of a pyridine solution containing 16.4 g of p-toluenesulfonyl chloride was added dropwise thereto under ice-cooling, followed by allowing to stand overnight. The reaction mixture was extracted with 300 mℓ of toluene, and the extract was washed with water and dried over magnesium sulfate. The solvent was removed by distillation to obtain 25 g of R-1-(p-toluenesulfonyloxy)-2-methyl-3-(2-tetrahydropyranyloxy)propane.

In 150 mℓ of THF was suspended 2.0 g of sodium hydride (55% in oil), and 200 mℓ of a THF solution containing 10 g of 4′-octyloxy-4-hydroxy-biphenyl was added to the suspension under ice-cooling. To the mixture was further added 300 mℓ of a dimethylformamide solution of 15 g of the above prepared R-1-(p-toluenesulfonyloxy)-2-methyl-3-(2-tetrahydropyranyloxy)propane, and the resulting mixture was stirred at 60°C for 5 hours. After allowing the mixture to cool, 500 mℓ of toluene was added thereto. The reaction mixture was washed successively with water and an alkali, and the solvent was removed by distillation. To the residue were added 300 mℓ of ethanol and 3 g of p-toluenesulfonic acid monohydr ate, followed by heating at 50°C for 1 hour. After allowing to cool, the crystals were allowed to grow under cooling. Filtration gave 11 g of R-4′-octyloxy-4-(2-methyl-3-hydroxypropoxy)biphenyl. A mixture of 1 g of this product, 0.4 g of butanoic acid, 1.0 g of N,N-dicyclohexylcarbodiimide, 0.1 g of 4-N,N-dimethylaminopyridine, and 30 mℓ of dichloromethane, was stirred at room temperature for 6 hours. The formed solid was separated by filtration, and the filtrate was washed successively with an acid, an alkali, and water, dried over magnesium sulfate, and distilled to remove the solvent. The residue was purified by chromatography using a column packed with active alumina. Recrystallization from ethanol yielded 0.7 g of S-4′-octyloxy-4-(2-methyl-3-butanoyloxy-propoxy)biphenyl having a melting point of from 73.6 to 74.4°C.

USE EXAMPLE 1

Liquid Crystal Composition A having the following formulation was prepared.

Formulation:

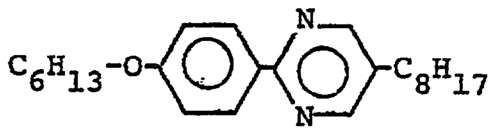

30%

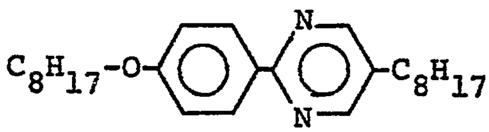

20%

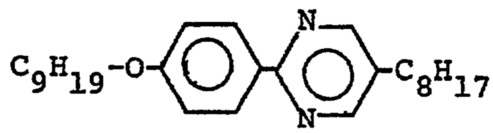

10%

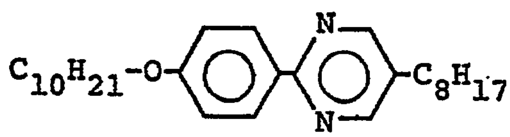

10%

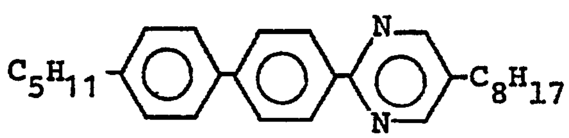

20%,

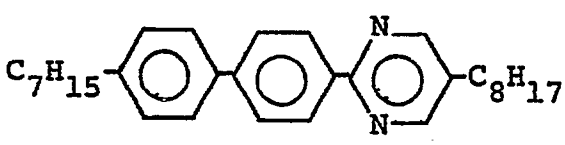

10%

Composition A was a non-chiral liquid crystal composition and had a crystal-smectic C phase transition point (C-SC point) of 4°C, a smectic C phase-smectic A phase transition point (SC-SA point) of 65°C, a smectic A phase-nematic phase transition point (SA-N point) of 79°C, and a nematic phase-isotropic phase transition point (N-I point) of 90°C.

Composition B comprising 80% of Composition A and 20% of the compound of Example 1 having formula:

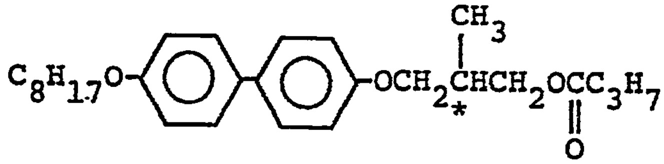

was a chiral smectic liquid crystal composition and had a chiral smectic C phase-smectic A phase transition point (SC*-SA point) of 58°C, a smectic A phase-cholesteric phase transition point (SA-Ch point) of 71.5°C, and a cholesteric phase-isotropic phase transition point (Ch-I point) of 79°C.

Composition B was introduced in a 2 µm thick cell equipped with transparent electrodes, which had been coated with polyvinyl alcohol as an aligning agent and had been subjected to parallel aligning treatment by rubbing the surface thereof, to thereby produce a liquid crystal display element. This element was placed between two polarizers crossed at right angles, and an electric field was applied thereto. Upon application of ±10 V, a change in transmission light intensity was observed. The response time was determined from the change in transmission light intensity, and the PS was determined according to a Sowyer-Tower method. The results obtained are shown in Table 2 below.

TABLE 2

| Temperature (°C) | Response Time (µsec) | PS (nC/cm²) |
|---|---|---|
| 55 | 78 | 1.4 |
| 45 | 110 | 2.4 |
| 35 | 150 | 3.3 |
| 25 | 250 | 3.8 |

As can be seen from the Table, use of the compound according to the present invention in a non-chiral smectic composition provides a satisfactory ferroelectric liquid crystal composition showing spontaneous polarization.

USE EXAMPLE 2

A nematic liquid crystal composition having the following formulation was prepared.

Formulation

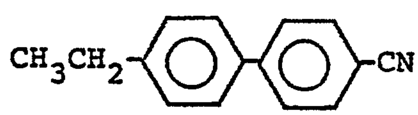
20%

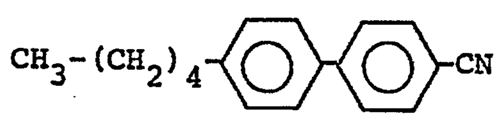
35%

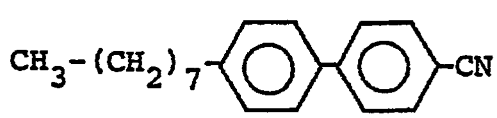
30%

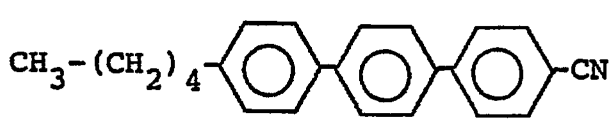
15%

The composition was introduced in a cell with transparent electrodes, the distance between the electrodes being 10 μm, which had been coated with polyvinyl alcohol as an aligning agent and had been subjected to parallel aligning treatment by rubbing the surface thereof, to thereby produce a TN type liquid crystal display element. Observation of the element under a polarization microscope showed that reverse twist domains were formed.

The compound of Example 1 was added to the above nematic liquid crystal composition in an amount of 1%. The resulting composition was introduced in the same cell as used above and observed as a TN type liquid crystal display element in the same manner as above. It was confirmed that the reverse twist domain disappeared, and a uniform nematic phase was observed.

USE EXAMPLE 3

A chiral nematic liquid crystal composition was prepared by adding 1% of the compound of Example 1 to ZLI-1132 (a trade name of a nematic liquid crystal composition on the market, produced by Merck & Co.), and its chiral pitch was measured by a Cano wedge method. As can be seen from the results shown in Table 3 below, the temperature dependence of the chiral pitch was small.

TABLE 3

| Temperature (°C) | Pitch (μm) |
|---|---|
| 20 | 29.6 |
| 30 | 31.0 |
| 40 | 32.6 |
| 50 | 34.4 |
| 60 | 36.4 |

While the invention has been described in detail and with reference to specific embodiments thereof, it will be apparent to one skilled in the art that various changes and modifications can be made therein without departing from the spirit and scope thereof.

**Claims**

1. A compound represented by formula (I)

$$R^1\text{-}A\text{-}B\text{-}O\text{-}CH_2\overset{CH_3}{\overset{|}{\underset{*}{C}}}HCH_2\text{-}O\text{-}R^2 \qquad (I)$$

wherein $R^1$ represents an alkyl group having 1 to 20 carbon atoms or an alkoxy group having from 1 to 20 carbon atoms, $R^2$ represents an alkyl group having from 1 to 15 carbon atoms, an alkanoyl group having from 2 to 15 carbon atoms, an alkoxycarbonyl group having from 2 to 15 carbon atoms, an alkoxyalkyl group having from 2 to 15 carbon atoms, an alkoxyalkanoyl group having from 3 to 15 carbon atoms, a halogenated alkyl group having from 1 to 15 carbon atoms or a halogenated alkanoyl group having from 2 to 15 carbon atoms, and the alkyl moiety in $R^1$ and $R^2$ is straight or branched; A and B each represents

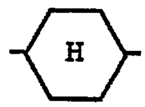, 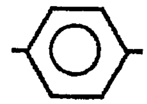, 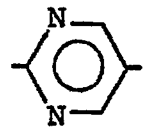,

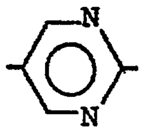, 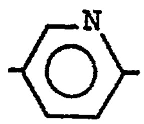, 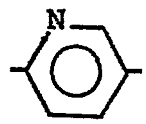, 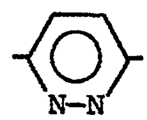, 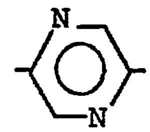,

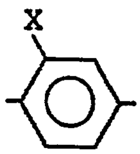 or 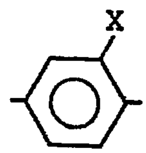,

wherein X represents a halogen atom, a cyano group or a trifluoromethyl group; and the asterisk mark * indicates that the carbon atom provided with * is an asymmetrical carbon atom.

2. The compound as claimed in Claim 1, wherein $R^1$ is an alkyl or alkoxy group having from 4 to 16 carbon atoms.

3. The compound as claimed in Claim 1, wherein $R^2$ is an alkyl, alkanoyl or alkoxyalkanoyl group having up to 10 carbon atoms.

4. The compound as claimed in Claim 1, wherein -A-B- is

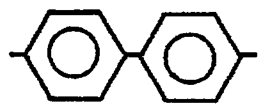, 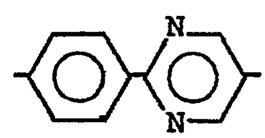,

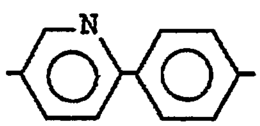, 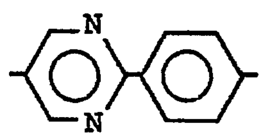, 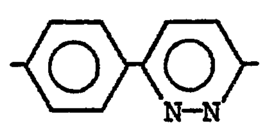 or

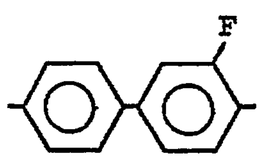.

5. A liquid crystal composition comprising at least one compound represented by formula (I)

$$R^1\text{-}A\text{-}B\text{-}O\text{-}CH_2\overset{CH_3}{\underset{*}{C}}HCH_2\text{-}O\text{-}R^2 \qquad (I)$$

wherein $R^1$ represents an alkyl group having 1 to 20 carbon atoms or an alkoxy group having from 1 to 20 carbon atoms, $R^2$ represents an alkyl group having from 1 to 15 carbon atoms, an alkanoyl group having from 2 to 15 carbon atoms, an alkoxycarbonyl group having from 2 to 15 carbon atoms, an alkoxyalkyl group having from 2 to 15 carbon atoms, an alkoxyalkanoyl group having from 3 to 15 carbon atoms, a halogenated alkyl group having from 1 to 15 carbon atoms or a halogenated alkanoyl group having from 2 to 15 carbon atoms, and the alkyl moiety in $R^1$ and $R^2$ is straight or branched; A and B each represents

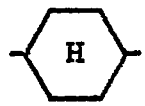, 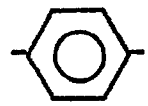, 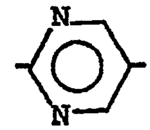,

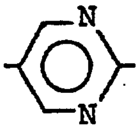, 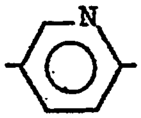, 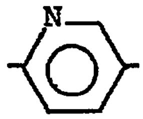, 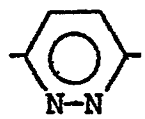, 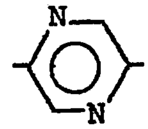,

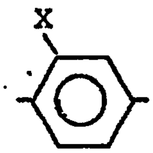 or 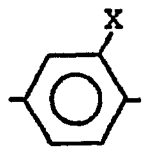,

wherein X represents a halogen atom, a cyano group or a trifluoromethyl group; and the asterisk mark * indicates that the carbon atom provided with * is an asymmetrical carbon atom.

6. The liquid crystal composition as claimed in Claim 5, wherein the composition exhibits a chiral smectic phase.

7. An optical switching element utilizing as a liquid crystal a ferroelectric liquid crystal composition comprising at least one compound represented by formula (I)

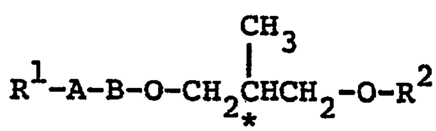 (I)

wherein $R^1$ represents an alkyl group having 1 to 20 carbon atoms or an alkoxy group having from 1 to 20 carbon atoms, $R^2$ represents an alkyl group having from 1 to 15 carbon atoms, an alkanoyl group having from 2 to 15 carbon atoms, an alkoxycarbonyl group having from 2 to 15 carbon atoms, an alkoxyalkyl group having from 2 to 15 carbon atoms, an alkoxyalkanoyl group having from 3 to 15 carbon atoms, a halogenated alkyl group having from 1 to 15 carbon atoms or a halogenated alkanoyl group having from 2 to 15 carbon atoms, and the alkyl moiety in $R^1$ and $R^2$ is straight or branched; A and B each represents

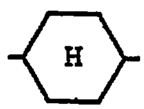, 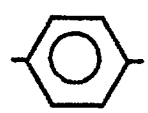, 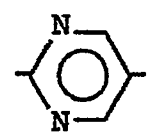,

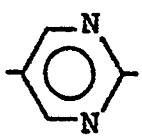, 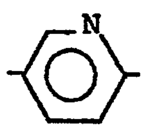, 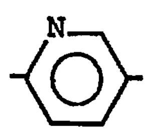, 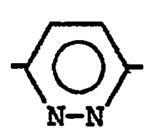, 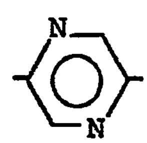,

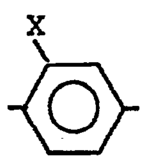 or 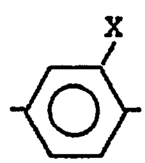,

wherein X represents a halogen atom, a cyano group or a trifluoromethyl group; and the asterisk mark * indicates that the carbon atom provided with * is an asymmetrical carbon atom.

**Patentansprüche**

1. Verbindung, dargestellt durch die Formel (I)

$$R^1-A-B-O-CH_2\overset{CH_3}{\underset{*}{C}}HCH_2-O-R^2 \qquad (I)$$

worin $R^1$ eine Alkyl-Gruppe mit 1 bis 20 Kohlenstoffatomen oder eine Alkoxy-Gruppe mit 1 bis 20 Kohlenstoffatomen, $R^2$ eine Alkyl-Gruppe mit 1 bis 15 Kohlenstoffatomen, eine Alkanoyl-Gruppe mit 2 bis 15 Kohlenstoffatomen, eine Alkoxycarbonyl-Gruppe mit 2 bis 15 Kohlenstoffatomen, eine Alkoxyalkyl-Gruppe mit 2 bis 15 Kohlenstoffatomen, eine Alkoxyalkanoyl-Gruppe mit 3 bis 15 Kohlenstoffatomen, eine halogenierte Alkyl-Gruppe mit 1 bis 15 Kohlenstoffatomen oder eine halogenierte Alkanoyl-Gruppe mit 2 bis 15 Kohlenstoffatomen bezeichnet, und der Alkyl-Anteil in $R^1$ und $R^2$ geradkettig oder verzweigt ist;

A und B jeweils 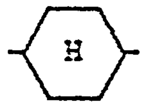, 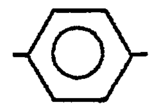, 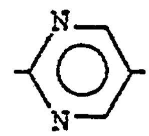,

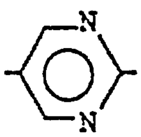, 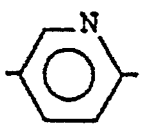, 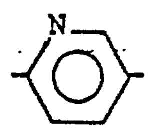, 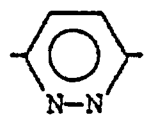, 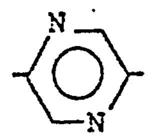,

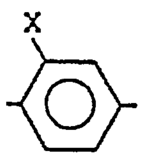 oder 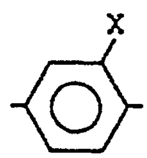, bezeichnen, wobei X ein Halogenatom, eine Cyano-Gruppe oder eine Trifluormethyl-Gruppe bezeichnet; und der Stern * das asymmetrische Kohlenstoffatom bezeichnet.

2. Verbindung gemäß Anspruch 1, in der $R^1$ eine Alkyl- oder Alkoxy-Gruppe mit 4 bis 16 Kohlenstoffatomen ist.

3. Verbindung gemäß Anspruch 1, in der $R^2$ eine Alkyl-, Alkanoyl- oder Alkoxyalkanoyl-Gruppe mit bis zu 10 Kohlenstoffatomen ist.

4. Verbindung gemäß Anspruch 1, in der -A-B-

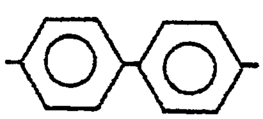, 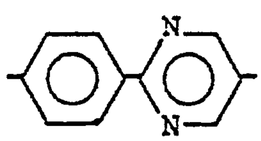, 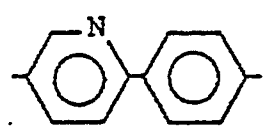,

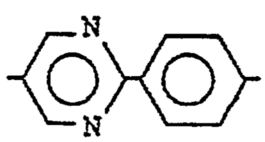, 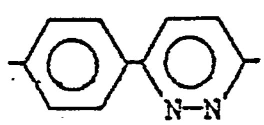 oder 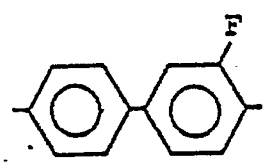

ist.

5. Flüssigkristall-Zusammensetzung, umfassend wenigstens eine Verbindung, dargestellt durch die Formel (I)

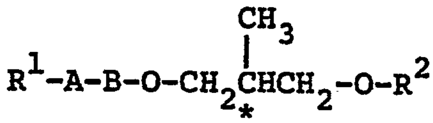 (I)

worin $R^1$ eine Alkyl-Gruppe mit 1 bis 20 Kohlenstoffatomen oder eine Alkoxy-Gruppe mit 1 bis 20 Kohlenstoffatomen, $R^2$ eine Alkyl-Gruppe mit 1 bis 15 Kohlenstoffatomen, eine Alkanoyl-Gruppe mit 2 bis 15 Kohlenstoffatomen, eine Alkoxycarbonyl-Gruppe mit 2 bis 15 Kohlenstoffatomen, eine Alkoxyalkyl-Gruppe mit 2 bis 15 Kohlenstoffatomen, eine Alkoxyalkanoyl-Gruppe mit 3 bis 15 Kohlenstoffatomen, eine halogenierte Alkyl-Gruppe mit 1 bis 15 Kohlenstoffatomen oder eine halogenierte Alkanoyl-Gruppe mit 2 bis 15 Kohlenstoffatomen bezeichnet, und der Alkyl-Anteil in $R^1$ und $R^2$ geradkettig oder verzweigt ist;

A und B jeweils 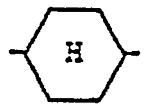, 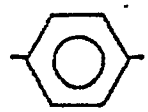, 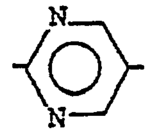,

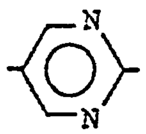, 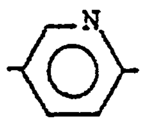, 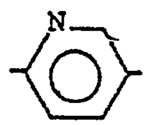, 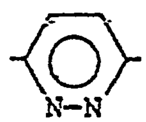, 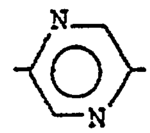,

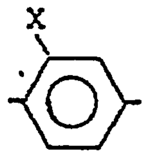 oder 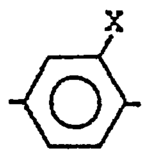, bezeichnet, wobei X ein Halogenatom, eine Cyano-Gruppe oder eine Trifluormethyl-Gruppe bezeichnet; und der Stern * das asymmetrische Kohlenstoffatom bezeichnet.

6. Flüssigkristall-Zusammensetzung gemäß Anspruch 5, in der die Zusammensetzung eine chiralesmektische Phase aufweist.

7. Optisches Schaltelement unter Verwendung einer ferroelektrischen Flüssigkristall-Zusammensetzung als Flüssigkristall, umfassend wenigstens eine Verbindung, dargestellt durch die Formel (I)

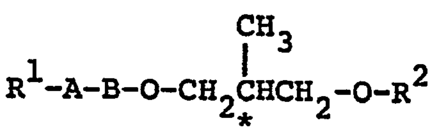 (I)

worin $R^1$ eine Alkyl-Gruppe mit 1 bis 20 Kohlenstoffatomen oder eine Alkoxy-Gruppe mit 1 bis 20 Kohlenstoffatomen, $R^2$ eine Alkyl-Gruppe mit 1 bis 15 Kohlenstoffatomen, eine Alkoanoyl-Gruppe mit 2 bis 15 Kohlenstoffatomen, eine Alkoxycarbonyl-Gruppe mit 2 bis 15 Kohlenstoffatomen, eine Alkoxyalkyl-Gruppe mit 2 bis 15 Kohlenstoffatomen, eine Alkoxyalkanoyl-Gruppe mit 3 bis 15 Kohlenstoffatomen, eine halogenierte Alkyl-Gruppe mit 1 bis 15 Kohlenstoffatomen oder eine halogenierte Alkanoyl-Gruppe mit 2 bis 15 Kohlenstoffatomen bezeichnet, und der Alkyl-Anteil in $R^1$ und $R^2$ geradkettig oder verzeigt ist;

A und B jeweils 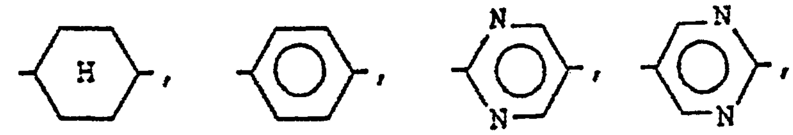

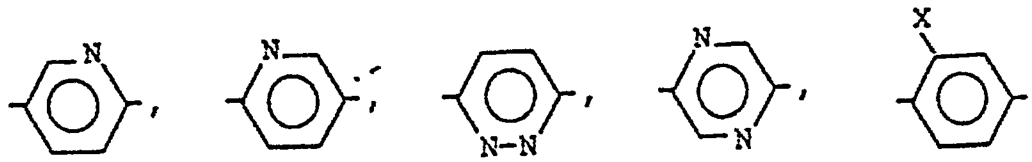

oder 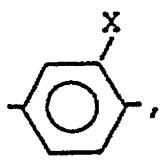 bezeichnet, wobei X ein Halogenatom, eine Cyano-Gruppe oder eine Trifluormethyl-Gruppe bezeichnet; und der Stern * das asymmetrische Kohlenstoffatom bezeichnet.

**Revendications**

1. Composé représenté par la formule (I)

$$R^1\text{-}A\text{-}B\text{-}O\text{-}CH_2\underset{*}{\overset{CH_3}{\overset{|}{C}}}HCH_2\text{-}O\text{-}R^2 \qquad (I)$$

dans laquelle $R^1$ représente un groupe alkyle ayant de 1 à 20 atomes de carbone ou un groupe alcoxy ayant de 1 à 20 atomes de carbone, $R^2$ représente un groupe alkyle ayant de 1 à 15 atomes de carbone, un groupe alcanoyle ayant de 2 à 15 atomes de carbone, un groupe alcoxycarbonyle ayant de 2 à 15 atomes de carbone, un groupe alcoxyalkyle ayant de 2 à 15 atomes de carbone, un groupe alcoxyalcanoyle ayant de 3 à 15 atomes de carbone, un groupe alkyle halogéné ayant de 1 à 15 atomes de carbone ou un groupe alcanoyle halogéné ayant de 2 à 15 atomes de carbone, et la partie alkyle dans $R^1$ et $R^2$ est linéaire ou ramifiée;

A et B représentent chacun 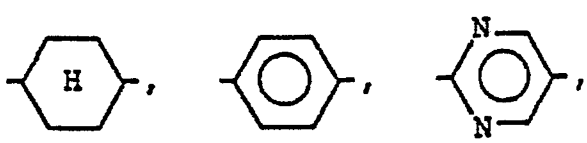

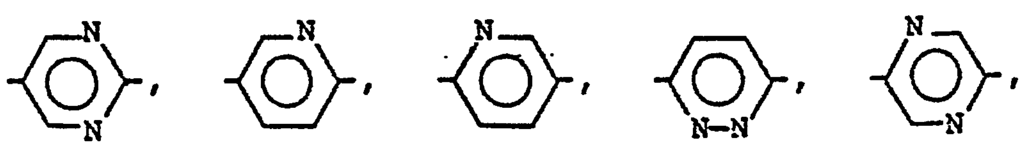

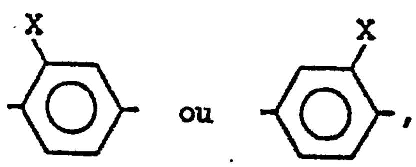

dans lesquels X représente un atome d'halogène, un groupe cyano ou un groupe trifluorométhyle; et le signe astérisque * indique que l' atome de carbone qui le porte est un atome de carbone asymétrique.

2. Composé tel que revendiqué dans la revendication 1, dans lequel $R^1$ est un groupe alkyle ou un groupe alcoxy ayant de 4 à 16 atomes de carbone.

3. Composé tel que revendiqué dans la revendication 1, dans lequel $R^2$ est un groupe alkyle, alcanoyle ou alcoxyalcanoyle ayant jusqu'à 10 atomes de carbone.

4. Composé tel que revendiqué dans la revendication 1, dans lequel -A-B- est

, 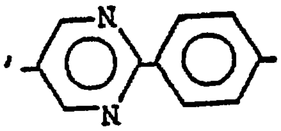 , 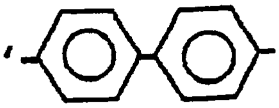

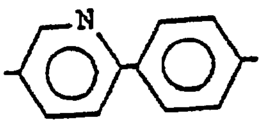 , 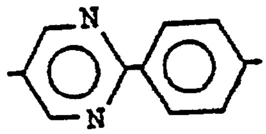 , 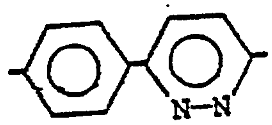 ou

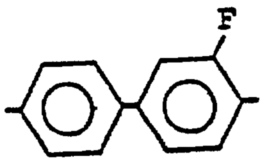 .

5. Composition de cristal liquide comprenant au moins un composé représenté par la formule (I)

$$R^1\text{-A-B-O-CH}_2\underset{*}{\overset{CH_3}{\overset{|}{C}}}\text{HCH}_2\text{-O-}R^2 \qquad (I)$$

dans laquelle $R^1$ représente un groupe alkyle ayant de 1 à 20 atomes de carbone ou un groupe alcoxy ayant de 1 à 20 atomes de carbone, $R^2$ représente un groupe alkyle ayant de 1 à 15 atomes de carbone, un groupe alcanoyle ayant de 2 à 15 atomes de carbone, un groupe alcoxycarbonyle ayant de 2 à 15 atomes de carbone, un groupe alcoxyalkyle ayant de 2 à 15 atomes de carbone, un groupe alcoxyalcanoyle ayant de 3 à 15 atomes de carbone, un groupe alkyle halogéné ayant de 1 à 15 atomes de carbone ou un groupe alcanoyle halogéné ayant de 2 à 15 atomes de carbone, et la partie alkyle dans $R^1$ et $R^2$ est linéaire ou ramifiée;

A et B représentent chacun 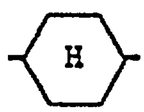 , 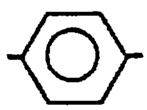 , 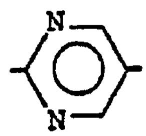 ,

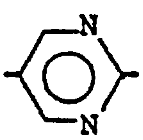 , 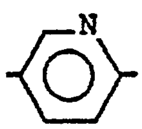 , 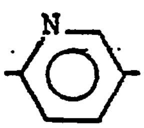 , 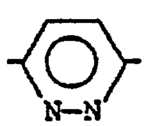 , 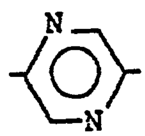 ,

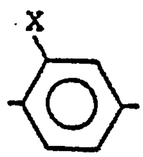 ou 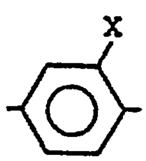 ,

dans lesquels X représente un atome d'halogène, un groupe cyano ou un groupe trifluorométhyle; et le signe astérisque * indique que l'atome de carbone qui le porte est un atome de carbone asymétrique.

6. Composition de cristal liquide telle que revendiquée dans la revendication 5, dans laquelle la composition présente une phase smectique chirale.

7. Elément optique de commutation utilisant en tant que cristal liquide une composition de cristal liquide ferro-électrique comprenant au moins un composé représenté par la formule (I)

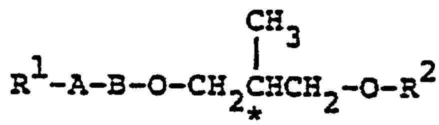

(I)

dans laquelle $R^1$ représente un groupe alkyle ayant de 1 à 20 atomes de carbone ou un groupe alcoxy ayant de 1 à 20 atomes de carbone, $R^2$ représente un groupe alkyle ayant de 1 à 15 atomes de carbone, un groupe alcanoyle ayant de 2 à 15 atomes de carbone, un groupe alcoxycarbonyle ayant de 2 à 15 atomes de carbone, un groupe alcoxyalkyle ayant de 2 à 15 atomes de carbone, un groupe alcoxyalcanoyle ayant de 3 à 15 atomes de carbone, un groupe alkyle halogéné ayant de 1 à 15 atomes de carbone ou un groupe alcanoyle halogéné ayant de 2 à 15 atomes de carbone, et la partie alkyle dans $R^1$ et $R^2$ est linéaire ou ramifiée;

A et B représentent chacun

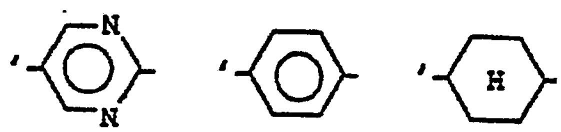

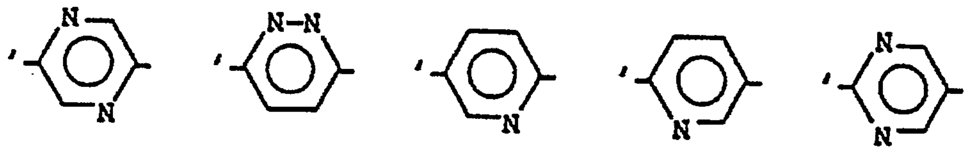

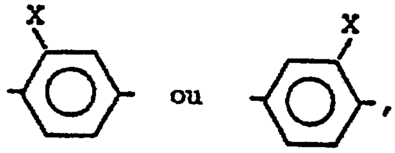

dans lesquels X représente un atome d'halogène, un groupe cyano ou un groupe trifluorométhyle; et le signe astérisque * indique que l'atome de carbone qui le porte est un atome de carbone asymétrique.